(19) **Europäisches Patentamt European Patent Office Office européen des brevets**

(11) **EP 4 752 793 A1**

(12) **EUROPEAN PATENT APPLICATION**
published in accordance with Art. 153(4) EPC

(43) Date of publication:
03.06.2026 Bulletin 2026/23

(21) Application number: 23955517.0

(22) Date of filing: 13.10.2023

(51) International Patent Classification (IPC):
*G06N 10/60* (2022.01) *G16C 10/00* (2019.01)

(52) Cooperative Patent Classification (CPC):
G06N 10/60; G16C 10/00

(86) International application number:
PCT/JP2023/037302

(87) International publication number:
WO 2025/079269 (17.04.2025 Gazette 2025/16)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(71) Applicant: QunaSys Inc.
Bunkyo-ku
Tokyo 113-0001 (JP)

(72) Inventors:
• NAKAGAWA, Yuya
Tokyo 113-0001 (JP)
• KAMOSHITA, Masahiko
Tokyo 113-0001 (JP)

(74) Representative: V.O.
P.O. Box 87930
2508 DH Den Haag (NL)

(54) **INFORMATION PROCESSING PROGRAM FOR QUANTUM COMPUTING, INFORMATION PROCESSING DEVICE FOR QUANTUM COMPUTING, INFORMATION PROCESSING METHOD FOR QUANTUM COMPUTING, AND INFORMATION PROCESSING SYSTEM FOR QUANTUM COMPUTING**

(57) An information processing device for quantum computing acquires an energy $E_k$ and a state vector $c_k$, which are results obtained by executing a quantum-selected configuration interaction (QSCI) algorithm on the basis of a Hamiltonian H and an input state of k-th step of a qubits, and sets an input state of a (k+1)-th step of the qubit on a basis of an energy expectation value according to the Hamiltonian H, the state vector $c_k$, and an operator P for each of operators P in a set of operators $\{P_1, P_2, \cdots, P_T\}$ set in advance.

FIG.4

## Description

Technical Field

The disclosed technology relates to an information processing program for quantum computing, an information processing device for quantum computing, an information processing method for quantum computing, and an information processing system for quantum computing.

Background Art

A task of obtaining an eigenvalue and an eigenstate of a matrix referred to as a "Hamiltonian of a quantum many-body system" that describes a state of a molecule or a substance is considered to be promising as an application destination of a quantum computer. Note that, the eigenvalue of the Hamiltonian corresponds to an energy eigenvalue of a molecule or a substance. Note that, the eigenstate of the Hamiltonian corresponds to an energy eigenstate of a molecule or a substance. The energy eigenstate is represented by a vector. Therefore, hereinafter, the vector representing the energy eigenstate may be simply referred to as a "state vector". In contrast, the energy eigenvalue is expressed by a scalar, and hereinafter, a scalar value representing the energy eigenvalue may be simply referred to as an "energy".

As an algorithm for computing the eigenvalue and eigenstate of the Hamiltonian using a quantum computer, Variational Quantum Eigensolver (for example, A. Peruzzo, J. McClean, P. Shadbolt, M.-H. Yung, X.-Q. Zhou, P. J. Love, A. Aspuru-Guzik, and J. L. O'Brien, "A variational eigenvalue solver on a photonic quantum processor", Nature Communications 5, 4213 (2014). (Literature 1)) is known.

However, in a case in which the quantum computer attempts to obtain the energy eigenvalue and the energy eigenstate using the Variational Quantum Eigensolver algorithm, the number of gates of a quantum circuit tends to be too large, and the number of times of measurement tends to be too large to obtain a computing result with sufficient accuracy.

Therefore, a method of efficiently computing the energy eigenvalue and the energy eigenstate using the quantum-selected configuration interaction algorithm (hereinafter, also simply referred to as a "QSCI algorithm") has been proposed (for example, K. Kanno, M. Kohda, R. Imai, S. Koh, K. Mitarai, W. Mizukami, and Y. O. Nakagawa, "Quantum-selected configuration interaction: classical diagonalization of hamiltonians in subspaces selected by quantum computers", arXiv preprint arXiv: 2302.11320 (2023). (Literature 2)).

In order to compute the energy eigenvalue and the energy eigenstate using the QSCI algorithm, it is necessary to set an input state of a qubit included in the quantum computer. However, in the Literature 2, an input state to be set for the qubit when computing the energy eigenvalue and energy eigenstate of the molecule or substance using the QSCI algorithm to efficiently and accurately compute the energy eigenvalue and energy eigenstate is not considered.

Therefore, in the related art, there has been a problem that it is not known which input state should be set for the qubit in order to efficiently and accurately compute the energy eigenvalue and the energy eigenstate using the QSCI algorithm.

SUMMARY OF INVENTION

Technical Problem

The disclosed technology has been made in view of the above circumstances, and provides an information processing program for quantum computing, an information processing device for quantum computing, an information processing method for quantum computing, and an information processing system for quantum computing capable of efficiently obtaining an input state of a qubit when computing an energy eigenvalue and an energy eigenstate using a QSCI algorithm.

Solution to Problem

In order to achieve the object, an information processing program for quantum computing of the disclosure is an information processing program for quantum computing to be executed by a computer, the quantum information processing program that causes the computer to execute processing including acquiring an energy $E_k$ and a state vector $c_k$, which are results obtained by executing a quantum-selected configuration interaction (QSCI) algorithm on the basis of a Hamiltonian H and an input state of k-th step of a qubits, and setting an input state of a (k+1)-th step of the qubit on the basis of an energy expectation value according to the Hamiltonian H, the state vector $c_k$, and an operator P for each of operators P in a set of operators $\{P_1, P_2, \cdots, P_T\}$ set in advance.

Advantageous Effects of Invention

According to the disclosed technology, an effect that an input state of a qubit can be efficiently obtained when an energy eigenvalue and an energy eigenstate are computed using a QSCI algorithm is obtained.

BRIEF DESCRIPTION OF DRAWINGS

Fig. 1 is a diagram illustrating an example of a schematic configuration of a hybrid system 100 of the present embodiment.
Fig. 2 is a schematic block diagram of a computer that functions as a server 110, a control device 121, and a user terminal 130.
Fig. 3 is a diagram for describing a QSCI algorithm.
Fig. 4 is a diagram illustrating an example of a sequence executed by the hybrid system 100 of the embodiment.
Fig. 5 is a diagram illustrating a result of a simulation experiment, which is an example.
Fig. 6 is a diagram illustrating a result of a simulation experiment, which is an example.

DESCRIPTION OF EMBODIMENTS

Hereinafter, an embodiment of the disclosed technology will be described in detail with reference to the drawings.

<Hybrid System 100 according to Embodiment>

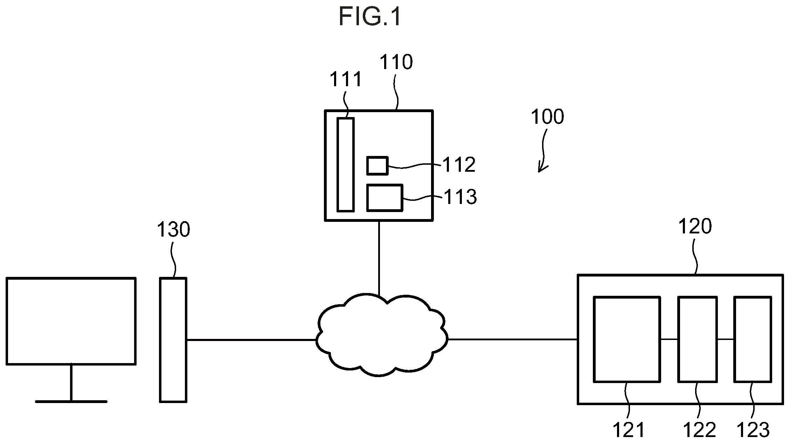

Fig. 1 illustrates a hybrid system 100 according to the embodiment. The hybrid system 100 is an example of an information processing system for quantum computing. The hybrid system 100 of the present embodiment includes a server 110, a quantum computer 120, and a user terminal 130 as an example of an information processing device for quantum computing. As illustrated in Fig. 1, the server 110, the quantum computer 120, and the user terminal 130 are connected via a computer network such as an IP network as an example. The hybrid system 100 is an example of a quantum information processing system.

In the hybrid system 100 of the embodiment, the quantum computer 120 performs predetermined quantum computing in response to a request from the server 110, and outputs a computing result of the quantum computing to the server 110. The server 110 outputs the computing result according to the quantum computing to the user terminal 130. As a result, predetermined computing processing is executed as an entire hybrid system 100.

The server 110 includes a communication unit 111 such as a communication interface, a processing unit 112 such as a processor and a central processing unit (CPU), and an information storage unit 113 including a storage device or a storage medium such as a memory and a hard disk, and is configured by executing an information processing program for quantum computing for performing each processing. Note that, the server 110 may include one or a plurality of devices or servers. The program may include one or a plurality of programs, and can be recorded in a computer-readable storage medium to form a non-transitory program product.

As an example, the quantum computer 120 generates an electromagnetic wave for irradiating at least any qubit of a qubit group 123 on the basis of information transmitted from the server 110. The quantum computer 120 executes a quantum circuit by irradiating at least any qubit of the qubit group 123 with the generated electromagnetic wave.

In an example in Fig. 1, the quantum computer 120 includes a control device 121 that communicates with the server 110, an electromagnetic wave generation device 122 that generates an electromagnetic wave in response to a request from the control device 121, and the qubit group 123 that receives electromagnetic wave irradiation from the electromagnetic wave generation device 122. The electromagnetic wave generation device 122 and the qubit group 123 in the quantum computer 120 also serve as a quantum processing unit (QPU). Note that, in the embodiment, the "quantum computer" does not mean not performing any operation using a classical bit, but refers to a computer including an operation using a qubit or a computer that simulates a behavior of a quantum computer. The computer that simulates a behavior of a quantum computer is, for example, a classical computer or the like that executes simulation or emulation of quantum computing. The "quantum computing" in the embodiment is computing performed by executing a quantum algorithm, and it is not essential to use a quantum computer as hardware. For example, the quantum computing is an operation using a quantum superposition state or an operation simulating an operation using a quantum superposition state. Therefore, the "quantum computing" in the embodiment is a concept including quantum computing by a quantum computer and simulation or emulation of quantum computing by a classical computer. The "qubit" in the embodiment is a concept including a qubit included in a quantum computer and a pseudo qubit when simulation or emulation of quantum computing by a classical computer is executed. A "qubit input state" in the embodiment is a concept including an input state for a qubit or an input state for an algorithm simulating a qubit.

The control device 121 is a classical computer that performs the operation using a classical bit, and alternatively performs a part of or an entire processing described in the present specification as being performed in the server 110.

The user terminal 130 is a classical computer that performs an operation using a classical bit. The user terminal 130 accepts information input from the user and executes processing according to the information.

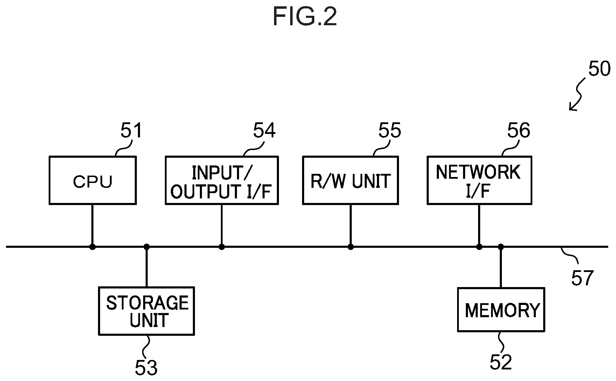

The server 110, the control device 121, and the user terminal 130 can be implemented by, for example, a computer 50 illustrated in Fig. 2. The computer 50 includes a central processing unit (CPU) 51, a memory 52 as a temporary storage area, and a nonvolatile storage unit 53. The computer 50 includes an input/output interface (I/F) 54 to which an external device, an output device and the like are connected, and a read/write (R/W) unit 55 that controls reading and writing of data with respect to a recording medium. The computer 50 includes a network I/F 56 connected to a network such as the Internet. The CPU 51, the memory 52, the storage unit 53, the input/output I/F 54, the R/W unit 55, and the network I/F 56 are connected to one another via a bus 57.

In the embodiment, a method of efficiently computing energy of a Hamiltonian H using a quantum-selected configuration interaction algorithm (hereinafter, also simply referred to as a "QSCI algorithm") disclosed in the following Cited Literatures 1 and 2 is proposed.

Cited Literature 1: K. Kanno, M. Kohda, R. Imai, S. Koh, K. Mitarai, W. Mizukami, and Y. O. Nakagawa, "Quantum-selected configuration interaction: classical diagonalization of hamiltonians in subspaces selected by quantum computers", arXiv preprint arXiv:2302.11320

(2023). Cited Literature 2: PCT/JP2022/026735

As disclosed in the Cited Literatures 1 and 2, the QSCI algorithm is also a method in which selected configuration interaction (selected CI), which is a computing method by a classical computer, is applied to a quantum computer. Selected CI is disclosed, for example, in the following Cited Literature 3.

Cited Literature 3: C. David Sherrill, Henry F. Schaefer III., "The Configuration Interaction Method: Advances in Highly Correlated Approaches", Advances in Quantum Chemistry, Volume 34, 1999, Pages 143-269

As disclosed in the Cited Literatures 1 and 2, the QSCI algorithm prepares an input state $|\Psi\rangle$ on a quantum computer and executes computing. However, a procedure of determining the input state $|\Psi\rangle$ is conventionally not known by which the energy of the Hamiltonian can be efficiently computed.

Therefore, in the embodiment, an algorithm capable of efficiently computing the energy of the Hamiltonian by adaptively changing the input state $|\Psi\rangle$ of the QSCI algorithm is proposed. Note that, a proposed method of the embodiment is also referred to as an ADAPT-QSCI algorithm because the input state $|\psi\rangle$ is adaptively changed. Note that, the "energy" and the "state vector" computed in the QSCI algorithm are not a true eigenvalue or a true eigenvector of the Hamiltonian to be computed, but are an eigenvalue and an eigenvector of a matrix obtained by approximating the Hamiltonian to be computed in a subspace.

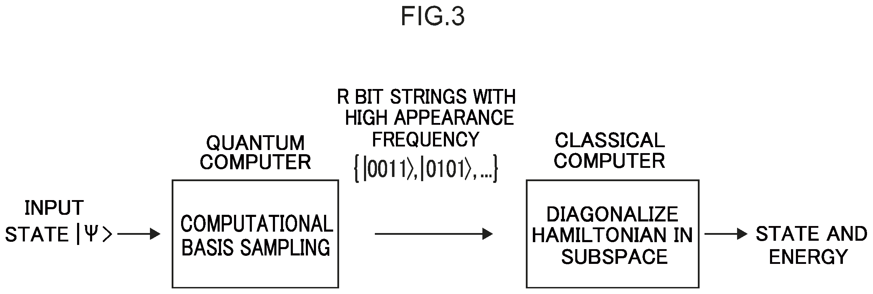

Fig. 3 is a diagram for describing an outline of the QSCI algorithm. In the QSCI algorithm, computing is executed on the basis of each piece of information of the Hamiltonian H to be computed, a quantum circuit U for setting the input state, the number of times of measurement (the number of shots) $N_s$, and an upper limit R of the number of computational bases.

As illustrated in Fig. 3, the QSCI algorithm executes each following computing.

1. A quantum computer generates an input state $|\psi\rangle = U|0\rangle$ and performs computational basis sampling measurement by the number of times of measurement $N_s$.

2. A classical computer selects R types of computational bases $|r_1\rangle, \cdots, |r_R\rangle$ with high frequency on the basis of a measurement result.

3. The classical computer generates an R×R-dimensional effective Hamiltonian $H^{(eff)}$ using R computational bases. Note that, each component of a matrix representing the effective Hamiltonian $H^{(eff)}$ is expressed by the following formula, in which a and b represent identification numbers for identifying the computational bases.

[Formula 1]

$$H_{ab}^{(\text{eff})} = \langle r_a | H | r_b \rangle$$

$$(1)$$

The classical computer computes an eigenvalue state and an eigenvalue by diagonalizing the matrix representing the effective Hamiltonian $H^{(eff)}$. A state vector c represented by the eigenvalue state and an energy E represented by the eigenvalue are output as a result.

Note that, the computational basis sampling is an operation that can be executed on an n-qubit quantum state |Ψ> generated on the quantum computer. When the computational basis sampling is executed in a case in which the quantum state can be expressed by the following formula, any of measured values (integers) i=0, 1, 2, ⋯, $2^n$-1 is obtained with a probability $|c_i|^2$. Note that, n is the total number of qubits.

[Formula 2]

$$|\psi\rangle = \sum_{i=0}^{2^n-1} c_i |i\rangle$$

(2)

Here, $c_i$ represents a complex number satisfying the following formula. Note that, i corresponds to the computational basis.

[Formula 3]

$$\sum_{i=0}^{2^n-1} |c_i|^2 = 1$$

(3)

Therefore, when the measurement is executed $N_s$ times, $N_s$ computational bases are obtained. For example, in the case of n=4 qubits, the computational bases are |0>=|0000>, |1>=|0001>, ⋯, |$2^n$-1>=|15>=|1111>.

In contrast to the QSCI algorithm, in the ADAPT-QSCI algorithm of the embodiment, a set of Pauli operators $\{P_1, P_2, \cdots P_T\}$ (hereinafter, also simply referred to as an "operator pool") is defined. In the ADAPT-QSCI algorithm, computing is executed on the basis of each piece of information of the Hamiltonian H to be computed, the quantum circuit U for setting the input state, an initial state, the number of times of measurement (the number of shots) $N_s$, and the upper limit R of the number of computational bases. The ADAPT-QSCI algorithm executes processing at the following steps 1 to 5.

1. A variable k representing repetition is set to k←0.

2. The quantum computer and the classical computer execute the QSCI algorithm. Specifically, the quantum computer and the classical computer set a k-th step input state $|\psi_k\rangle$ and execute an $N_s$-shot QSCI algorithm. As a result, a combination of a state vector $c_k$ and an energy $E_k$ is obtained. Note that, in a case in which the energy $E_k$ converges, the ADAPT-QSCI algorithm is stopped.

3. The classical computer computes, for each of operators P in the operator pool $\{P_1, P_2, \cdots P_T\}$, a differential of an energy expectation value expressed by the following formula. The differential of the energy expectation value expressed by the following formula is a differential of an energy expectation value at θ=0 of a variational state $e^{i\theta P}|c_k\rangle$ in the R-dimensional subspace specified in the execution of a k-th QSCI algorithm. Note that, θ represents a rotation angle of a rotation gate, and H represents the Hamiltonian.

[Formula 4]

$$\langle c_k | i(HP - PH) | c_k \rangle$$

(4)

Note that, $|c_k\rangle$ is obtained by interpreting the R-dimensional state vector $c_k$ as an n-qubit state ($2^n$-dimensional vector). Specifically, $|c_k\rangle$ is expressed by the following formula. $(c_k)_l$ is an 1-th component of the state vector $c_k$. $|r_1^{(k)}\rangle$ is an 1-th computational basis selected in the ADAPT-QSCI algorithm of a k-th repetition step.

[Formula 5]

$$|c_k\rangle = \sum_{l=1}^{R}(c_k)_l \left| r_l^{(k)} \right\rangle$$

(5)

$<c_k|i(HP\text{-}PH)|c_k>$ expressed in Formula (4) described above is a differential value of an energy expectation value $E_\Psi(\theta)=<\Psi(\theta)|H|\Psi(\theta)>$ in a case in which the input state $|\psi(\theta)>$ is $e^{i\theta P}|c_k>$ and $\theta=0$ as described in the following formula. In the ADAPT-QSCI algorithm, at step 4 to be described later, an operator having the largest absolute value of the differential value of the energy expectation value is selected, so that an operator having a smaller energy expectation value is efficiently selected.
[Formula 6]

$$\frac{dE_\Psi(\theta=0)}{d\theta} = \langle c_k|i(HP-PH)|c_k\rangle$$

(6)

In the case of considering matrix display in the R-dimensional subspace of i(HP-PH) that can be regarded as an operator, $<c_k|i(HP\text{-}PH)|c_k>$ represented in Formula (4) described above can be computed by polynomial time of R using the classical computer. Therefore, in a case in which R set in the QSCI algorithm is not exponentially large with respect to a qubit number n, the classical computer can efficiently compute $<c_k|i(HP\text{-}PH)|c_k>$ expressed in Formula (4) described above. (HP-PH) that can be regarded as the operator can also be computed by polynomial time by the classical computer.
4. The classical computer selects an operator $P_{n_k}(1 \leq n_k \leq T)$ that maximizes an absolute value of a differential of an energy expectation value expressed by the following Formula (7). The classical computer computes $\theta_k^*$ that minimizes an energy expectation value expressed by the following Formula (8). The energy expectation value expressed by the following Formula (8) is an energy expectation value of a variational state $e^{i\theta k P_{nk}}|c_k>$ in the R-dimensional subspace specified by the k-th step QSCI algorithm.
[Formula 7]

$$|\langle c_k|i(HP-PH)|c_k\rangle|$$

(7)

$$\langle c_k|e^{-i\theta_k P_{n_k}} H e^{i\theta_k P_{n_k}}|c_k\rangle$$

(8)

Note that, the following Formula (9) holds from a property of the Pauli operator. Note that, I represents an identity operator. The following Formula (10) holds. The following Formula (11) is derived from the following Formulae (9) and (10).
[Formula 8]

$$P_{n_k}^2 = I$$

(9)

$$e^{i\theta_k P} = \cos\theta_k\, I + i\sin\theta_k\, P$$

(10)

$$e^{-i\theta_k P_{n_k}} H e^{i\theta_k P_{n_k}}$$

$$= \cos^2\theta_k\, H + \sin^2\theta_k\, P_{n_k} H P_{n_k} + i\sin\theta_k \cos\theta_k\, (HP_{n_k} - P_{n_k}H)$$

(11)

The classical computer can efficiently compute the following Formula (8) by using matrix display in the R-dimensional subspace of each of H, $P_{nk}HP_{nk}$, and $HP_{nk}$-$P_{nk}$ in Formula (11) described above. Therefore, it is possible to search for $\theta_k^*$ that minimizes Formula (8) described above using only the classical computer.

5. The classical computer sets a new input state $|\psi_{k+1}\rangle$ as expressed in the following Formula (12). The classical computer returns to the processing at step 2 while setting k←k+1. The new input state $|\psi_{k+1}\rangle$ is used in the computing of the quantum computer at next step 2.

[Formula 9]

$$|\psi_{k+1}\rangle = e^{i\theta_k^* P_{n_k}} |\psi_k\rangle$$

(12)

By executing the processing at each step described above, the state vector c and the energy E are acquired as computing results. Note that, in a case in which the ADAPT-QSCI algorithm stops at k=m, a state vector $c_m$ and the energy $E_m$ are acquired as computing results. At that time, the input state is expressed by the following formula.

[Formula 10]

$$|\psi_m\rangle = e^{i\theta_{m-1}^* P_{n_{m-1}}} \dots e^{i\theta_0^* P_{n_0}} |\psi_0\rangle$$

(13)

Note that, as can be seen from the new input state $|\psi_m\rangle$ in the case of k=m, the ADAPT-QSCI algorithm sequentially adds a rotation gate $e^{i\theta k * Pnk}$, which is an operator that minimizes the energy expectation value represented in Formula (8) described above, to the quantum circuit, and the quantum circuit sets the new input state $|\psi_{k+1}\rangle$.

[Operation of Hybrid System 100 of Embodiment]

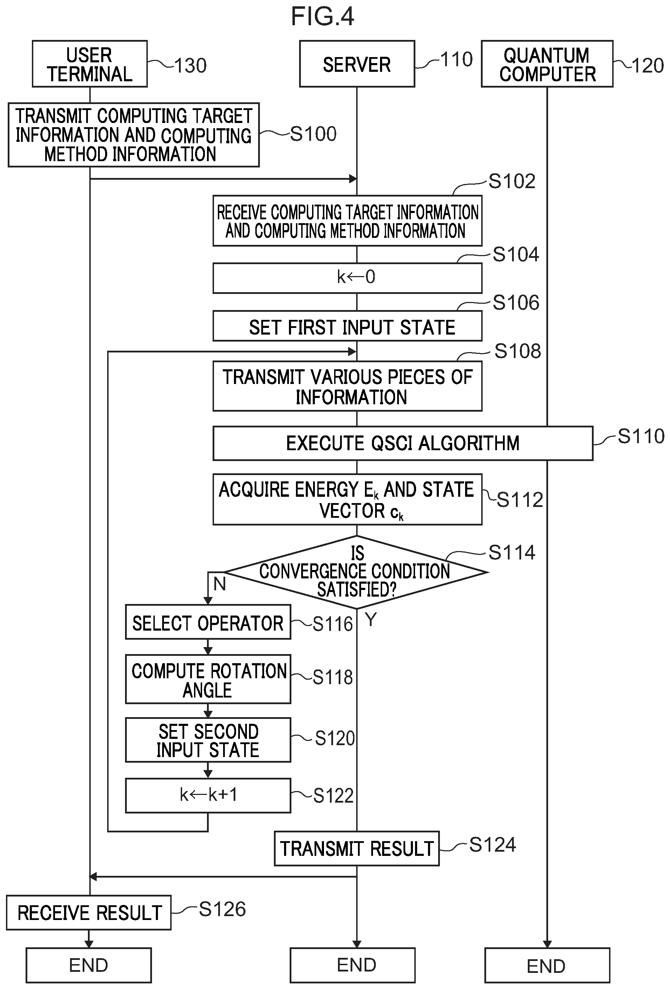

A specific operation of the hybrid system 100 of the embodiment is described. In each device of the hybrid system 100, each processing illustrated in Fig. 4 is executed.

First, at step S100, the user terminal 130 transmits, to the server 110, computing target information that is information regarding a computing target and computing method information that is information regarding a computing method, which are input from the user.

The computing target information includes, for example, information regarding the operator pool $\{P_1, P_1, \cdots, P_T\}$ to be computed and the Hamiltonian H to be computed and the like. The computing method information includes, for example, each information of the quantum circuit U for setting the input state, the initial state of the qubit, the number of shots $N_s$, and the upper limit R of the number of computational bases and the like.

Next, at step S102, the server 110 receives the computing target information and the computing method information transmitted from the user terminal 130.

At step S104, the server 110 sets a repetition variable k to 0.

At step S106, the server 110 sets an initial state of the qubit. Specifically, the server 110 generates a quantum circuit in which an initial state is prepared on the quantum computer 120.

At step S108, the server 110 transmits, to the quantum computer 120, the quantum circuit generated at step S104 or the quantum circuit generated at step S120 described later, and various pieces of information necessary for execution of the QSCI algorithm received at step S102.

At step S110, a known QSCI algorithm is executed between the server 110 and the quantum computer 120.

At step S112, the server 110 acquires the state vector $c_k$ and the energy $E_k$, which are computing results of the QSCI algorithm executed at step 110.

At step S114, the server 110 determines whether or not a convergence condition set in advance is satisfied. In a case where the convergence condition is satisfied, the process proceeds to step S124. In contrast, in a case in which the convergence condition is not satisfied, the process proceeds to step S116.

As the convergence condition, various conditions can be set. For example, as the convergence condition, a condition regarding whether or not a difference between the energy $E_k$ computed at a k-th repetition step and an energy $E_{k+1}$ computed at a (k+1)-th repetition step satisfies a predetermined threshold condition can be adopted. Note that, as the threshold condition in this case, for example, a condition that a difference between the energy $E_k$ computed at the k-th repetition step and the energy $E_{k+1}$ computed at the (k+1)-th repetition step is a threshold set in advance or smaller or smaller than a threshold set in advance can be adopted. Alternatively, for example, as the convergence condition, a condition regarding whether or not an absolute value $|<c_k|i(HP-PH)|c_k>|$ of the differential computed at the k-th repetition step satisfies a predetermined threshold condition can be adopted. Note that, as the threshold condition in this case, for example, a condition that the absolute value $|<c_k|i(HP-PH)|c_k>|$ of the differential computed at the k-th repetition step is a threshold set in advance or smaller or smaller than a threshold set in advance can be adopted.

At step S116, the server 110 sets the input state $|\psi_{k+1}>$ of the qubit to be used in the next execution of the QSCI algorithm on the basis of the energy expectation value according to the Hamiltonian H, the state vector $c_k$, and the operator P for each of the operators P in the operator pool $\{P_1, P_2, \cdots, Pr\}$.

Specifically, first, at step S116, the server 110 computes, for each of the operators P in the operator pool $\{P_1, P_2, \cdots, P_T\}$, an absolute value $|<c_k|i(HP-PH)|c_k>|$ of a differential of an energy expectation value $<c_k|e^{-i\theta P}He^{i\theta P}|c_k>$.

At step S116, the server 110 selects the operator $P_{nk}$ that maximizes the absolute value $|<c_k|i(HP-PH)|c_k>|$ of the computed differential among the operators P included in the operator pool $\{P_1, P_2, \cdots, P_T\}$.

At step S118, the server 110 computes the rotation angle $\theta_k^*$ of the operator $P_{nk}$ that minimizes the energy expectation value expressed by Formula (8) described above on the basis of the operator $P_{nk}$ selected at step S116. Note that, this computing is executed on the basis of a known method.

At step S120, the server 110 sets the input state $|\psi_{k+1}>$ at the (k+1)-th repetition step expressed by Formula (12) described above on the basis of the operator $P_{nk}$ selected at step S116 and the rotation angle $\theta_k^*$ computed at step S118.

Specifically, at step S120, the server 110 adds the operator $P_{nk}$, which is the rotation gate having the rotation angle $\theta_k^*$, to the quantum circuit set at the k-th repetition step, thereby setting the quantum circuit for setting the input state $|\psi_{k+1}>$ used in the next execution of the QSCI algorithm. In a next repetition loop, the QSCI algorithm is executed using the newly set quantum circuit.

At step S122, the server 110 sets a repetition variable k to k+1.

At step S124, the server 110 transmits the energy $E_k$ and the state vector $c_k$ obtained at step S112 to the user terminal 130.

At step S126, the user terminal 130 receives the result transmitted from the server 110.

As described above, the server in the hybrid system of the embodiment acquires the energy $E_k$ and the state vector $c_k$, which are results obtained by executing the QSCI algorithm on the basis of the Hamiltonian H and the input state of a k-th step of the qubit. The server sets the input state of a (k+1)-th step of the qubit on the basis of the energy expectation value according to the Hamiltonian H, the state vector $c_k$, and the operator P for each of the operators P in the set of operators $\{P_1, P_2, \cdots, P_T\}$ set in advance. Note that, the energy expectation value for each of the operators P in the set of operators $\{P_1, P_2, \cdots, P_T\}$ set in advance is the energy expectation value in the R-dimensional subspace defined by the QSCI algorithm on the basis of the Hamiltonian H and the input state of the qubit. As a result, when an energy eigenvalue and an energy eigenstate of a molecule or a substance are computed using the QSCI algorithm, the input state of the qubit that can efficiently and accurately compute the energy eigenvalue and the energy eigenstate can be efficiently obtained.

A conventional problem in the QSCI algorithm is that a simple method of generating an input state of a qubit has not been studied. Therefore, in the embodiment, as described above, the QSCI algorithm is repeatedly executed, and the input state of the qubit at that time is adaptively improved. This makes it possible to execute quantum computing with a smaller number of shots while reducing the number of gates in the quantum circuit when acquiring the energy of a quantum many-body system by the quantum computing.

Specifically, in the embodiment, as described above, the operator pool serving as a generator of the rotation gate for setting the input state is set in advance. In the embodiment, the best operator is selected from the operator pool, and the rotation gate corresponding to the operator is added to the quantum circuit. The selection of the operator and the determination of the rotation angle can be executed only by classical computing using the state vector $c_k$ obtained in the QSCI algorithm. At that time, since the quantum computer only repeatedly executes simple sampling measurement for the QSCI algorithm, computing resources required for the quantum computing can be greatly reduced.

More specifically, the server in the hybrid system of the embodiment repeatedly executes the setting of the input state of the qubit and the acquisition of the energy $E_k$ and the state vector $c_k$. At that time, the server selects the operator $P_{nk}$ from the operator pool $\{P_1, P_2, \cdots, P_T\}$ that maximizes the absolute value $|<c_k|i(HP-PH)|c_k>|$ of the differential of the energy expectation value $<c_k|e^{-i\theta P}He^{i\theta P}|c_k>$. Next, the server computes the rotation angle $\theta_k^*$ of the operator $P_{nk}$ that minimizes the energy expectation value expressed by Formula (8) described above on the basis of the selected operator $P_{nk}$. The server sets the input state $|\psi_{k+1}>$ at the (k+1)-th repetition step expressed by Formula (12) described above on the basis of the selected operator $P_{nk}$ and the computed rotation angle $\theta_k^*$. The newly set input state $|\psi_{k+1}>$ is prepared on the quantum computer 120 when executing the QSCI algorithm in the (k+1)-th repetition step. In this manner, in order to prepare a state in which the energy expectation value is as small as possible on the quantum computer, the server selects the operator $P_{nk}$ that maximizes the absolute value of the differential of the energy expectation value, and computes the rotation angle $\theta_k^*$ of the operator $P_{nk}$ so that the energy expectation value is minimized. By selecting the operator $P_{nk}$ that maximizes the absolute value of the differential of the energy expectation value, the operator $P_{nk}$ that is estimated to have the largest reduction in the energy expectation value is selected, and the input state for obtaining the energy of the Hamiltonian can be efficiently searched for.

Note that, the server adds the operator $P_{nk}$, which is the rotation gate having the rotation angle $\theta_k^*$, to the quantum circuit set at the (k-1)-th repetition step, thereby setting the quantum circuit for setting the input state of the qubit. The QSCI algorithm is executed using the newly set quantum circuit. As a result, the quantum circuit that generates the input state for obtaining the energy of the Hamiltonian can be efficiently constructed.

Note that, the technology of the present disclosure is not limited to the above-described embodiment, and various modifications and applications can be made without departing from the gist of the invention.

Hereinafter, modified examples of the embodiment will be described.

[Modified Example 1]

In the embodiment, the case where the operator $P_{nk}$ is first selected from the operator pool $\{P_1, P_2, \cdots, P_T\}$ and then the rotation angle $\theta_k^*$ of the operator $P_{nk}$ is determined has been described as an example, but the invention is not limited thereto. For example, a combination of the operator $P_{nk}$ and the rotation angle $\theta_k^*$ that minimizes the energy expectation value expressed by Formula (8) described above may be simultaneously computed, and the input state $|\psi_{k+1}>$ at the (k+1)-th repetition step expressed by Formula (12) described above may be set. Note that, as a method of simultaneously computing the combination of the operator $P_{nk}$ and the rotation angle $\theta_k^*$ that minimizes the energy expectation value expressed by Formula (8) described above, a known method can be adopted. For example, the method disclosed in the following Cited Literature 4 may be adopted.

Cited Literature 4: Ken M. Nakanishi, Keisuke Fujii, and Synge Todo, "Sequential minimal optimization for quantum-classical hybrid algorithms", Phys. Rev. Research 2, Published 29 October 2020, <https://journals.aps.org/prresearch/abstract/10.1103/PhysRevResearch.2.043158>

[Modified Example 2]

In the above-described embodiment, the case where one operator $P_{nk}$ is selected from the operator pool $\{P_1, P_2, \cdots, P_T\}$ and then the rotation angle $\theta_k^*$ of the operator $P_{nk}$ is determined has been described as an example, but the invention is not limited thereto. A plurality of operators $P_{n1k}, \cdots, P_{nsk}$ ($ns_k$ is an operator identification number) may be selected from the operator pool $\{P_1, P_2, \cdots, P_T\}$, and rotation angles $\theta_{1k}^*, \cdots, \theta_{sk}^*$ ($s_k$ is a rotation angle identification number) of the plurality of operators $P_{n1k}, \cdots, P_{nsk}$ may be computed to set the input state $|\psi_{k+1}>$ in the (k+1)-th repetition step. In this case, for example, the server first selects a plurality of operators $P_{n1k}, \cdots, P_{nsk}$ ($ns_k$ is an operator identification number) in descending order of the absolute value $|<c_k|i(HP-PH)|c_k>|$ of the differential of the energy expectation value $<c_k|e^{-i\theta P}He^{i\theta P}|c_k>$ from the operator pool $\{P, P_2, \cdots, P_T\}$. The server computes the rotation angles $\theta_{1k}^*, \cdots, \theta_{sk}^*$ of the plurality of operators $P_{n1k}, \cdots, P_{nsk}$ that minimize the energy expectation value expressed by the following Formula (14) on the basis of the plurality of selected operators $P_{n1k}, \cdots, P_{nsk}$. The server sets the input state $|\psi_{k+1}>$ at the (k+1)-th repetition step expressed by the following Formula (15) on the basis of the plurality of selected operators $P_{n1k}, \cdots, P_{nsk}$ and the computed rotation angles $\theta_{1k}^*, \cdots, \theta_{sk}^*$.

$$\langle c_k | e^{-i\theta_{s_k}P_{ns_k}} \cdots e^{-i\theta_{1_k}P_{n1_k}} H e^{i\theta_{1_k}P_{n1_k}} \cdots e^{i\theta_{s_k}P_{ns_k}} | c_k \rangle$$

$$(14)$$

$$|\psi_{k+1}\rangle = e^{i\theta^*_{1_k}P_{n1_k}} \cdots e^{i\theta^*_{s_k}P_{ns_k}} |\psi_k\rangle$$

$$(15)$$

[Modified Example 3]

In Modified Example 2, the case where the rotation angles of the plurality of operators are determined after selecting the plurality of operators has been described as an example, but the invention is not limited thereto. For example, combinations of a plurality of operators and their rotation angles may be computed simultaneously. In this case, for example, the server computes combinations of a plurality of operators $P_{n1k}, \cdots, P_{nsk}$ and rotation angles $\theta_{1k}{}^*, \cdots, \theta_{sk}{}^*$ that minimize the energy expectation value expressed by Formula (14) described above. The server sets the input state $|\psi_{k+1}\rangle$ at the (k+1)-th repetition step expressed by Formula (15) described above on the basis of the plurality of operators $P_{n1k}, \cdots, P_{nsk}$ and rotation angles $\theta_{1k}{}^*, \cdots, \theta_{sk}{}^*$.

[Modified Example 4]

In the embodiment, the case where the energy $E_k$ and the state vector $c_k$ at the k-th repetition step are output as the results in the case where the convergence condition is satisfied as an example, but the invention is not limited thereto. For example, in a case in which the convergence condition is satisfied at the k-th repetition step, the server may output an output value according to an energy $E_{k-1}$ obtained at the (k-1)-th repetition step or the energy $E_k$ obtained at the k-th repetition step, and an output value according to a state vector $c_{k-1}$ obtained at the (k-1)-th repetition step or the state vector $c_k$ obtained at the k-th repetition step. In this case, for example, the server may output an average of a result obtained at the (k-1)-th repetition step and the result obtained at the k-th repetition step as a final result. Alternatively, the server may output any of the result obtained at the (k-1)-th repetition step and the result obtained at the k-th repetition step as the final result.

[Modified Example 5]

In the embodiment, the case where the operator in the operator pool is the Pauli operator has been described as an example, but the invention is not limited thereto. The operator in the operator pool may be other types of operators.

[Modified Example 6]

In the embodiment, the case of computing the energy eigenvalue and the energy eigenstate of the Hamiltonian H has been described as an example, but the energy eigenvalue and the energy eigenstate include the energy eigenvalue and the energy eigenstate corresponding to a ground state and the energy eigenvalue and the energy eigenstate corresponding to an excited state. In the embodiment, the case of computing the energy eigenvalue and the energy eigenstate corresponding to the ground state has been described as an example, but it is also possible to compute the energy eigenvalue and the energy eigenstate corresponding to the excited state. In the case of computing the energy eigenvalue and the energy eigenstate corresponding to the excited state, for example, it is possible to compute the energy eigenvalue and the energy eigenstate corresponding to the excited state by combining the method disclosed in the Cited Literature 1 with the proposed method in the embodiment.

[Modified Example 7]

In the embodiment, the case where the server 110 executes all of the various pieces of processing executed by the classical computer has been described as an example, but the invention is not limited thereto. For example, the server 110 may execute some of the various pieces of processing executed by the classical computer, and another classical computer different from the server 110 may execute other processing. For example, another classical computer different from the server 110 may execute the processing of the QSCI algorithm at step S110 illustrated in Fig. 4, and the server 110 may execute each processing at steps S102 to S108 and steps S112 to S124 illustrated in Fig. 4.

In the embodiment, transmission and reception of information between the server 110 and the quantum computer 120 may be performed in any manner. For example, transmission and reception of a parameter of the quantum circuit, transmission and reception of a measurement result and the like between the server 110 and the quantum computer 120 may be sequentially performed every time predetermined computing is completed, or may be performed after all the computings are completed.

In the embodiment, the case where the computing target information is transmitted from the user terminal 130 to the server 110 and the server 110 executes computing according to the computing target information has been described as an example, but the invention is not limited thereto. The user terminal 130 may transmit the computing target information to the server 110 or a storage medium or a storage device accessible by the server 110 via a computer network such as an IP network, or the computing target information may be stored in the storage medium or the storage device and delivered to an operator of the server 110, and the operator may input the computing target information to the server 110 using the storage medium or the storage device.

In the embodiment, the case where the quantum circuit is executed by the irradiation of the electromagnetic wave has been described as an example, but the invention is not limited thereto, and the quantum circuit may be executed by a different method.

In the embodiment, the case where the quantum computer 120 executes quantum computing has been described as an example, but the invention is not limited thereto. For example, the quantum computing may be executed by a classical computer that simulates the behavior of the quantum computer.

In the embodiment, the case is assumed that the server 110 and the quantum computer 120 are managed by different organizations, but the server 110 and the quantum computer 120 may be integrally managed by the same organization. In this case, it is conceivable that the control device 121 of the quantum computer 120 plays the role of the server 110 in the above description.

Note that, in the embodiment, if there is no description of "only" such as "on the basis of only ××", "according to only ××", or "in the case of only xx", it should be noted that it is assumed that additional information can be considered in the specification. As an example, the description of "perform b in the case of a" does not necessarily mean "always perform b in the case of a" unless explicitly stated otherwise.

In the embodiment, in a case in which expressions such as "optimize" or "optimized parameter" are used, it should be noted that these expressions of "optimization" mean bringing closer to an optimal state. Therefore, in the case of obtaining a parameter that minimizes a certain function, it should be noted that the parameter obtained by optimizing the function is assumed to be not a global solution that minimizes the function but a local solution in some cases. In a case in which the expression of "maximize" or "minimize" is used, it should be noted that the expression of "maximize" or "minimize" also includes the meaning of bringing the state close to a "maximized" state or "minimized state".

Even if there is an aspect of performing an operation different from the operation described in the specification in some method, program, terminal, device, server, or system (hereinafter "method or the like"), each aspect of the disclosed technology is directed to the same operation as any of the operations described in the specification, and the presence of an operation different from the operation described in the specification does not make the method or the like out of the scope of each aspect of the technology of the present disclosure.

In the specification of the present application, the embodiment in which the program is installed in advance has been described, but the program can be provided by being stored in a computer-readable recording medium.

Note that, the processing executed by the CPU reading software (program) in the embodiment may be executed by various processors other than the CPU. Examples of the processor in this case include a programmable logic device (PLD) in which a circuit configuration can be changed after manufacturing such as a field-programmable gate array (FPGA), a dedicated electric circuit that is a processor having a circuit configuration exclusively designed for executing specific processing such as an application specific integrated circuit (ASIC) and the like. Each processing may be executed by one of these various processors, or may be executed by a combination of two or more processors of the same type or different types (for example, a plurality of FPGAs, a combination of a CPU and an FPGA and the like). More specifically, a hardware structure of these various processors is an electric circuit in which circuit elements such as semiconductor elements are combined.

In each of the embodiments, the aspect in which the program is stored (installed) in advance in the storage has been described, but the present invention is not limited thereto. The program may be provided in a form stored in a non-transitory storage medium such as a compact disk read only memory (CD-ROM), a digital versatile disk read only memory (DVD-ROM), and a universal serial bus (USB) memory. The program may be downloaded from an external device via a network.

Each processing of the embodiment may be configured by a computer, a server or the like including a general-purpose arithmetic processing device, a storage device or the like, and each processing may be executed by a program. This program is stored in a storage device, and can be recorded in a recording medium such as a magnetic disk, an optical disk, or a semiconductor memory, or can be provided through a network. Of course, any other components need not be implemented by a single computer or server, and may be implemented in a distributed manner in a plurality of computers connected by a network.

Each component of the hybrid system of the embodiment need not be implemented by a single computer or server, and may be implemented in a distributed manner in a plurality of computers connected by a network.

For example, the processing executed by the classical computer of the embodiment may be performed by a plurality of classical computers connected via a network in a distributed manner. For example, the processing executed by the quantum computer of each embodiment may be performed by a plurality of quantum computers connected via a network in a distributed manner. In this case, a hybrid system is configured by at least one or more classical computers and at least one or more quantum computers.

[Example]

Next, an example of the ADAPT-QSCI algorithm of the embodiment will be described. In the example, a comparison between the ADAPT-QSCI algorithm and an ADAPT-VQE algorithm, which is a known method, was performed. The ADAPT-VQE algorithm is disclosed in the following Cited Literatures 5 and 6.

Cited Literature 5: H. R. Grimsley, S. E. Economou, E. Barnes, and N. J. Mayhall, "An adaptive variational algorithm for exact molecular simulations on a quantum computer", Nature Communications 10, 10.1038/s41467-019-10988-2 (2019).

Cited Literature 6: H. L. Tang, V. Shkolnikov, G. S. Barron, H. R. Grimsley, N. J. Mayhall, E. Barnes, and S. E. Economou, "Qubit-adapt-vqe: An adaptive algorithm for constructing hardware-efficient ansatze on a quantum processor", PRX Quantum 2, 020310 (2021).

In this simulation, numerical simulation was performed on a $H_4$ molecule in which four hydrogens are arranged in a straight line at a distance of 1Å. Note that, a quantum chemical Hamiltonian was generated by performing Hartree-Fock method using a known STO-3G basis, and the Hamiltonian H was obtained using Jordan-Wigner transformation. As an initial state, $|\psi_0\rangle=|00001111\rangle$ (Hartree-Fock state) was used. An operator pool P' was defined as follows.

$$P^{'}=\{X_{2i}Y_{2j}, X_{2i+1}Y_{2j+1}|i,j=0,1,2,3$$

are different integers and i<j}

U $\{X_pX_qX_rY_s, X_pX_qY_rX_s, X_pY_qX_rX_s, Y_pX_qX_rX_s|0\leq p,q,r,s\leq 7$ are different integers and p+q+r+s=0 mod 2, p<q<r<s} where $X_m$ and $Y_m$ are Pauli X and Y operators on an m-th qubit.

The number of shots $N_S$ was $10^5$, and an upper limit R of the number of computational bases in the QSCI algorithm was R=14. Numerical simulation was performed on ADAPT-VQE and ADAPT-QSCI using the same conditions described above. Note that, in the ADAPT-VQE, numerical simulation using a strict energy expectation value not considering random shot fluctuation was performed. In the ADAPT-QSCI, numerical simulation was performed in consideration of random shot fluctuations, and a result of executing the ADAPT-QSCI algorithm 10 times from the same initial state was recorded.

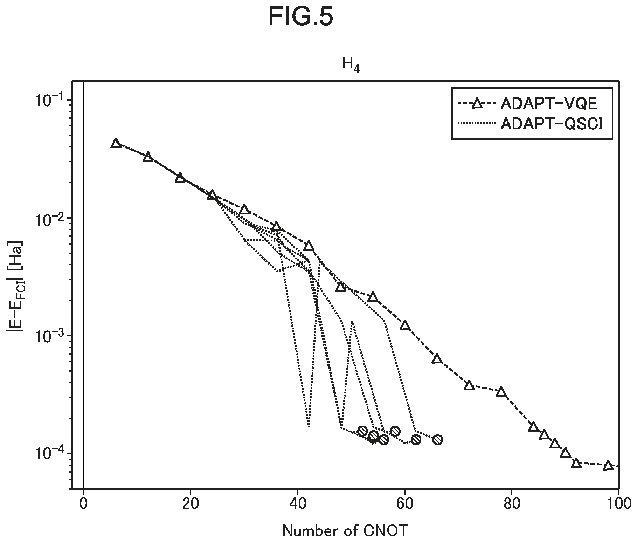

Fig. 5 is a simulation result of the $H_4$ molecule. In Fig. 5, the horizontal axis represents the number of CNOT gates required to generate the state $|\psi k\rangle$ of each step, and the vertical axis represents a difference between an output energy E of each algorithm and a strict ground state energy $E_{FCI}$. Note that, the number of CNOT gates was computed using a standard decomposition into CNOT and one-qubit gates as introduced in the following Cited Literature 7. In the numerical simulation of the ADAPT-QSCI, convergence was determined when an energy difference between repetition steps was $10^{-5}$ hartree or less. In Fig. 5, the energy at the time of convergence is indicated by dots. Regarding a numerical simulation result of the ADAPT-QSCI in Fig. 5, a graph terminated by one circle corresponds to a result of one numerical simulation.

Cited Literature 7: Abhinav Anand, et al, "A quantum computing view on unitary coupled cluster theory", Chemical Society Reviews, Issue 5, 2022, <https://pubs.rsc.org/en/content/articlelanding/2022/CS/D1 CS00932J>

Referring to Fig. 5, it can be seen that the ADAPT-QSCI can compute energy with higher accuracy in a state with a smaller number of CNOT gates than ADAPT-VQE. Since the number of gates is smaller in the ADAPT-QSCI, it can be said that the ADAPT-QSCI is more easily executed by a noisy intermediate-scale quantum (NISQ) device.

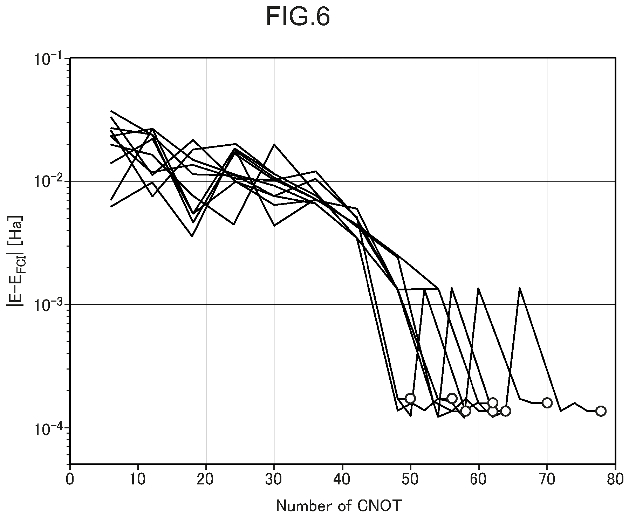

Fig. 6 is a result of numerical simulation performed to verify noise resistance of the ADAPT-QSCI. In Fig. 6 also, a graph terminated by one circle corresponds to a result of one numerical simulation. This numerical simulation is a numerical simulation performed on the $H_4$ molecule using the same condition as that of the numerical simulation in Fig. 5 when there is noise. Note that, in this numerical simulation, a noise model using a depolarization noise channel in which an error rate between a two-qubit gate and a measurement operation is 1% was used. An error reduction method disclosed in the following Cited Literature 8 and 9 was used. Referring to Fig. 6, it can be seen that the ADAPT-QSCI can output energy with high accuracy with an error of $10^{-3}$ hartree or less also in a situation with noise.

Cited Literature 8: Tudor Giurgica-Tiron, et al, "Digital zero noise extrapolation for quantum error mitigation", <https://ieeexplore.ieee.org/document/9259940>
Cited Literature 9: Filip B. Maciejewski, et al, "Mitigation of readout noise in near-term quantum devices by classical post-processing based on detector tomography", <https://quantum-journal.org/papers/q-2020-04-24-257/>

All documents, patent applications, and technical standards described in this specification are incorporated herein by reference to the same extent as if each document, patent application, and technical standard were specifically and individually described to be incorporated by reference.

(Appendix)

Aspects of the disclosure will be appended below.

(Appendix 1)

An information processing program for quantum computing to be executed by a computer,
the information processing program for quantum computing that causes the computer to execute processing including:

acquiring an energy $E_k$ and a state vector $c_k$, which are results obtained by executing a quantum-selected configuration interaction (QSCI) algorithm on the basis of a Hamiltonian H and an input state of a k-th step of qubits; and
setting an input state of a (k+1)-th step of the qubit on the basis of an energy expectation value according to the Hamiltonian H, the state vector $c_k$, and an operator P for each of operators P in a set of operators $\{P_1, P_2, \cdots, P_T\}$ set in advance.

(Appendix 2)

The information processing program for quantum computing according to Appendix 1, in which
the energy expectation value for each of the operators P in the set of operators $\{P_1, P_2, \cdots, P_T\}$ set in advance is an energy expectation value in an R-dimensional subspace defined by the QSCI algorithm on the basis of the Hamiltonian H and the input state of the qubit.

(Appendix 3)

The information processing program for quantum computing according to Appendices 1 or 2, including:

further acquiring an energy $E_{k+1}$ and a state vector $c_{k+1}$ obtained by executing the QSCI algorithm on the basis of the Hamiltonian H and the input state of the (k+1)-th step;
determining whether the energy $E_k$ and the energy $E_{k+1}$ satisfy a convergence condition;
outputting an energy based on the energy $E_k$ and the energy $E_{k+1}$ in a case in which the convergence condition is satisfied; and
setting an input state of a (k+2)-th step of the qubit on the basis of an energy expectation value according to the Hamiltonian H, the state vector $c_{k+1}$, and the operator P for each of the operators P in the set of operators $\{P_1, P_2, \cdots, P_T\}$ set in advance in a case in which the convergence condition is not satisfied.

(Appendix 4)

The information processing program for quantum computing according to Appendix 3, in which

the energy expectation value of the k-th step is a value obtained by $<c_k|e^{-i\theta P}He^{i\theta P}|c_k>$ (where i represents a computational basis obtained in the QSCI algorithm, and $\theta$ represents a rotation angle of an operator),
when setting an input state $|\psi_{k+1}>$ of the (k+1)-th step, an operator $P_{nk}$ (n is an operator identification number) that maximizes an absolute value $|<c_k|i(HP-PH)|c_k>|$ of a differential of the energy expectation value $<c_k|e^{-i\theta P}He^{i\theta P}|c_k>$ is selected from the set of operators $\{P_1, P_2, \cdots, P_T\}$, a rotation angle $\theta_k^*$ of the operator $P_{nk}$ that minimizes an energy expectation value expressed by the following Formula (A) is computed on the basis of the selected operator $P_{nk}$, and the input state $|\psi_{k+1}>$ of the (k+1)-th step expressed by the following Formula (B) is set on the basis of the selected operator $P_{nk}$ and the computed rotation angle $\theta_k^*$, and
the energy $E_{k+1}$ and the state vector obtained by executing the QSCI algorithm on the basis of Hamiltonian H and

the input state $|\psi_{k+1}\rangle$ of the (k+1)-th step are acquired.

[Formula 11]

$$\langle c_k | e^{-i\theta_k P_{n_k}} H e^{i\theta_k P_{n_k}} | c_k \rangle$$

(A)

[Formula 12]

$$|\psi_{k+1}\rangle = e^{i\theta_k^* P_{n_k}} |\psi_k\rangle$$

(B)

(Appendix 5)

The information processing program for quantum computing according to Appendix 3, in which

the energy expectation value of the k-th step is a value obtained by $\langle c_k | e^{-i\theta P} H e^{i\theta P} | c_k \rangle$ (where i represents a computational basis obtained in the QSCI algorithm, and $\theta$ represents a rotation angle of an operator),

when setting the input state $|\psi_{k+1}\rangle$ of the (k+1)-th step, a combination of an operator $P_{nk}$ (n is an operator identification number) and a rotation angle $\theta_k^*$ that minimizes an energy expectation value expressed by the following Formula (A) is computed, and the input state $|\psi_{k+1}\rangle$ of the (k+1)-th step expressed by the following Formula (B) is set, and

the energy $E_{k+1}$ and the state vector $c_{k+1}$ obtained by executing the QSCI algorithm on the basis of the Hamiltonian H and the input state $|\psi_{k+1}\rangle$ in the (k+1)-th step are acquired.

[Formula 13]

$$\langle c_k | e^{-i\theta_k P_{n_k}} H e^{i\theta_k P_{n_k}} | c_k \rangle$$

(A)

[Formula 14]

$$|\psi_{k+1}\rangle = e^{i\theta_k^* P_{n_k}} |\psi_k\rangle$$

(B)

(Appendix 6)

The information processing program for quantum computing according to Appendix 3, in which

the energy expectation value of the k-th step is a value obtained by $\langle c_k | e^{-i\theta P} H e^{i\theta P} | c_k \rangle$ (where i represents a computational basis obtained in the QSCI algorithm, and $\theta$ represents a rotation angle of an operator),

when setting the input state $|\psi_{k+1}\rangle$ of the (k+1)-th step, a plurality of operators $P_{n1k}, \cdots, P_{nsk}$ ($ns_k$ is an operator identification number) is selected from the set of operators $\{P_1, P_2, \cdots, P_T\}$ in descending order of an absolute value $|\langle c_k | i(HP-PH) | c_k \rangle|$ of the differential of the energy expectation value $\langle c_k | e^{-i\theta P} H e^{i\theta P} | c_k \rangle$, rotation angles $\theta_{1k}^*, \cdots, \theta_{sk}^*$ ($s_k$ is a rotation angle identification number) of the plurality of operators $P_{n1k}, \cdots, P_{nsk}$ that minimize an energy expectation value expressed by the following Formula (C) are computed on the basis of the selected plurality of operator $P_{n1k}, \cdots, P_{nsk}$, and the input state $|\psi_{k+1}\rangle$ in the (k+1)-th step expressed by the following Formula (D) is set on the basis of the selected plurality of operators $P_{n1k}, \cdots, P_{nsk}$ and the computed rotation angles $\theta_{1k}^*, \cdots, \theta_{sk}^*$, and

the energy $E_{k+1}$ and the state vector $c_{k+1}$ obtained by executing the QSCI algorithm on the basis of the Hamiltonian H

and the input state $|\psi_{k+1}\rangle$ in the (k+1)-th step are acquired.
[Formula 15]

$$\langle c_k | e^{-i\theta_{s_k} P_{ns_k}} \cdots e^{-i\theta_{1_k} P_{n1_k}} H e^{i\theta_{1_k} P_{n1_k}} \cdots e^{i\theta_{s_k} P_{ns_k}} | c_k \rangle$$

(C)

[Formula 16]

$$|\psi_{k+1}\rangle = e^{i\theta_{1_k}^* P_{n1_k}} \cdots e^{i\theta_{s_k}^* P_{ns_k}} |\psi_k\rangle$$

(D)

(Appendix 7)

The information processing program for quantum computing according to Appendix 3, in which

the energy expectation value of the k-th step is a value obtained by $<c_k|e^{-i\theta P}He^{i\theta P}|c_k>$ (where i represents a computational basis obtained in the QSCI algorithm, and $\theta$ represents a rotation angle of an operator),
when setting the input state $|\psi_{k+1}\rangle$ of the (k+1)-th step, a combination of a plurality of operators $P_{n1_k}, \cdots, P_{nsk}$ ($ns_k$ is an operator identification number) and rotation angles $\theta_{1_k}^*, \cdots, \theta_{sk}^*$ ($s_k$ is a rotation angle identification number) that minimizes an energy expectation value expressed by the following Formula (C) is computed, the input state $|\psi_{k+1}\rangle$ in the (k+1)-th step expressed by the following Formula (D) is set on the basis of the plurality of operators $P_{n1_k}, \cdots, P_{nsk}$ and the rotation angles $\theta_{1_k}^*, \cdots, \theta_{sk}^*$, and
the energy $E_{k+1}$ and the state vector $c_{k+1}$ obtained by executing the QSCI algorithm on the basis of the Hamiltonian H and the input state $|\psi_{k+1}\rangle$ in the (k+1)-th step are acquired.
[Formula 17]

$$\langle c_k | e^{-i\theta_{s_k} P_{ns_k}} \cdots e^{-i\theta_{1_k} P_{n1_k}} H e^{i\theta_{1_k} P_{n1_k}} \cdots e^{i\theta_{s_k} P_{ns_k}} | c_k \rangle$$

(C)

[Formula 18]

$$|\psi_{k+1}\rangle = e^{i\theta_{1_k}^* P_{n1_k}} \cdots e^{i\theta_{s_k}^* P_{ns_k}} |\psi_k\rangle$$

(D)

(Appendix 8)

The information processing program for quantum computing according to Appendix 4, in which
the convergence condition is whether or not a difference between the energy $E_k$ computed in the k-th step and the energy $E_{k+1}$ computed in the (k+1)-th step satisfies a threshold condition.

(Appendix 9)

The information processing program for quantum computing according to Appendix 4, in which
the convergence condition is whether or not the absolute value $|<c_k|i(HP-PH)|c_k>|$ of the differential computed in the k-th step satisfies a predetermined value condition.

(Appendix 10)

The information processing program for quantum computing according to any one of Appendices 4 to 9, in which

a quantum circuit for setting the input state is set by adding the operator $P_{nk}$ that is a rotation gate having the rotation angle $\theta_k{}^*$ to the quantum circuit set in the k-th step, and
the QSCI algorithm is executed using the quantum circuit.

(Appendix 11)

An information processing device for quantum computing that

acquires an energy $E_k$ and a state vector $c_k$, which are results obtained by executing a quantum-selected configuration interaction (QSCI) algorithm on the basis of a Hamiltonian H and an input state of a k-th step of qubits; and
sets an input state of a (k+1)-th step of the qubit on the basis of an energy expectation value according to the Hamiltonian H, the state vector $c_k$, and an operator P for each of operators P in a set of operators $\{P_1,P_2,\cdots,P_T\}$ set in advance.

(Appendix 12)

An information processing system for quantum computing that

acquires an energy $E_k$ and a state vector $c_k$, which are results obtained by executing a quantum-selected configuration interaction (QSCI) algorithm on the basis of a Hamiltonian H and an input state of a k-th step of qubits; and
sets an input state of a (k+1)-th step of the qubit on the basis of an energy expectation value according to the Hamiltonian H, the state vector $c_k$, and an operator P for each of operators P in a set of operators $\{P_1,P_2,\cdots,P_T\}$ set in advance.

## Claims

1. An information processing program for quantum computing to be executed by a computer,

   the information processing program for quantum computing that causes the computer to execute processing comprising:
   acquiring an energy $E_k$ and a state vector $c_k$, which are results obtained by executing a quantum-selected configuration interaction (QSCI) algorithm on a basis of a Hamiltonian H and an input state of a k-th step of qubits; and
   setting an input state of a (k+1)-th step of the qubit on a basis of an energy expectation value according to the Hamiltonian H, the state vector $c_k$, and an operator P for each of operators P in a set of operators $\{P_1,P_2,\cdots,P_T\}$ set in advance.

2. The information processing program for quantum computing according to claim 1, wherein
   the energy expectation value for each of the operators P in the set of operators $\{P_1,P_2,\cdots,P_T\}$ set in advance is an energy expectation value in an R-dimensional subspace defined by the QSCI algorithm on a basis of the Hamiltonian H and the input state of the qubit.

3. The information processing program for quantum computing according to claim 1 or 2, comprising:

   further acquiring an energy $E_{k+1}$ and a state vector $c_{k+1}$ obtained by executing the QSCI algorithm on a basis of the Hamiltonian H and the input state of the (k+1)-th step;
   determining whether the energy $E_k$ and the energy $E_{k+1}$ satisfy a convergence condition;
   outputting an energy based on the energy $E_k$ and the energy $E_{k+1}$ in a case in which the convergence condition is satisfied; and
   setting an input state of a (k+2)-th step of the qubit on a basis of an energy expectation value according to the Hamiltonian H, the state vector $c_{k+1}$, and the operator P for each of the operators P in the set of operators $\{P_1,P_2,\cdots,P_T\}$ set in advance in a case in which the convergence condition is not satisfied.

4. The information processing program for quantum computing according to claim 3, wherein

the energy expectation value of the k-th step is a value obtained by $<c_k|e^{-i\theta P}He^{i\theta P}|c_k>$ (where i represents a computational basis obtained in the QSCI algorithm, and $\theta$ represents a rotation angle of an operator),

when setting an input state $|\psi_{k+1}>$ of the (k+1)-th step, an operator $P_{nk}$ (n is an operator identification number) that maximizes an absolute value $|<c_k|i(HP-PH)|c_k>|$ of a differential of the energy expectation value $<c_k|e^{-i\theta P}He^{i\theta P}|c_k>$ is selected from the set of operators $\{P_1,P_2,\cdots,P_T\}$, a rotation angle $\theta_k^*$ of the operator $P_{nk}$ that minimizes an energy expectation value expressed by following Formula (A) is computed on a basis of the selected operator $P_{nk}$, and the input state $|\psi_{k+1}>$ of the (k+1)-th step expressed by following Formula (B) is set on a basis of the selected operator $P_{nk}$ and the computed rotation angle $\theta_k^*$, and

the energy $E_{k+1}$ and the state vector obtained by executing the QSCI algorithm on a basis of the Hamiltonian H and the input state $|\psi_{k+1}>$ of the (k+1)-th step are acquired.

[Formula 1]

$$\left\langle c_k \left| e^{-i\theta_k P_{n_k}} H e^{i\theta_k P_{n_k}} \right| c_k \right\rangle$$

(A)

[Formula 2]

$$|\psi_{k+1}\rangle = e^{i\theta_k^* P_{n_k}} |\psi_k\rangle$$

(B)

5. The information processing program for quantum computing according to claim 3, wherein

the energy expectation value of the k-th step is a value obtained by $<c_k|e^{-i\theta P}He^{i\theta P}|c_k>$ (where i represents a computational basis obtained in the QSCI algorithm, and $\theta$ represents a rotation angle of an operator),

when setting the input state $|\psi_{k+1}>$ of the (k+1)-th step, a combination of an operator $P_{nk}$ (n is an operator identification number) and a rotation angle $\theta_k^*$ that minimizes an energy expectation value expressed by following Formula (A) is computed, and the input state $|\psi_{k+1}>$ of the (k+1)-th step expressed by following Formula (B) is set, and

the energy $E_{k+1}$ and the state vector $c_{k+1}$ obtained by executing the QSCI algorithm on a basis of the Hamiltonian H and the input state $|\psi_{k+1}>$ in the (k+1)-th step are acquired.

[Formula 1]

$$\left\langle c_k \left| e^{-i\theta_k P_{n_k}} H e^{i\theta_k P_{n_k}} \right| c_k \right\rangle$$

(A)

[Formula 2]

$$|\psi_{k+1}\rangle = e^{i\theta_k^* P_{n_k}} |\psi_k\rangle$$

(B)

6. The information processing program for quantum computing according to claim 3, wherein

the energy expectation value of the k-th step is a value obtained by $<c_k|e^{-i\theta P}He^{i\theta P}|c_k>$ (where i represents a computational basis obtained in the QSCI algorithm, and $\theta$ represents a rotation angle of an operator),

when setting the input state $|\psi_{k+1}\rangle$ of the (k+1)-th step, a plurality of operators $P_{n1k},\cdots,P_{nsk}$ ($ns_k$ is an operator identification number) is selected from the set of operators $\{P_1,P_2,\cdots,P_T\}$ in descending order of an absolute value $|\langle c_k|i(HP-PII)|c_k\rangle|$ of the differential of the energy expectation value $\langle c_k|e^{-i\theta P}He^{i\theta P}|c_k\rangle$, rotation angles $\theta_{1k}^{*},\cdots,\theta_{sk}^{*}$ ($s_k$ is a rotation angle identification number) of the plurality of operators $P_{n1k},\cdots,P_{nsk}$ that minimize an energy expectation value expressed by following Formula (C) are computed on a basis of the selected plurality of operator $P_{n1k},\cdots,P_{nsk}$, and the input state $|\psi_{k+1}\rangle$ in the (k+1)-th step expressed by following Formula (D) is set on a basis of the selected plurality of operators $P_{n1k},\cdots,P_{nsk}$ and the computed rotation angles $\theta_{1k}^{*},\cdots,\theta_{sk}^{*}$, and the energy $E_{k+1}$ and the state vector $c_{k+1}$ obtained by executing the QSCI algorithm on a basis of the Hamiltonian H and the input state $|\psi_{k+1}\rangle$ in the (k+1)-th step are acquired.

[Formula 1]

$$\left\langle c_k \middle| e^{-i\theta_{s_k}P_{ns_k}} \cdots e^{-i\theta_{1_k}P_{n1_k}} H e^{i\theta_{1_k}P_{n1_k}} \cdots e^{i\theta_{s_k}P_{ns_k}} \middle| c_k \right\rangle$$

(C)

[Formula 2]

$$\left|\psi_{k+1}\right\rangle = e^{i\theta_{1_k}^{*}P_{n1_k}} \cdots e^{i\theta_{s_k}^{*}P_{ns_k}} \left|\psi_k\right\rangle$$

(D)

7. The information processing program for quantum computing according to claim 3, wherein

the energy expectation value of the k-th step is a value obtained by $\langle c_k|e^{-i\theta P}He^{i\theta P}|c_k\rangle$ (where i represents a computational basis obtained in the QSCI algorithm, and $\theta$ represents a rotation angle of an operator),
when setting the input state $|\psi_{k+1}\rangle$ of the (k+1)-th step, a combination of a plurality of operators $P_{n1k},\cdots,P_{nsk}$ ($n_{Sk}$ is an operator identification number) and rotation angles $\theta_{1k}^{*},\cdots,\theta_{sk}^{*}$ ($s_k$ is a rotation angle identification number) that minimizes an energy expectation value expressed by following Formula (C) is computed, the input state $|\psi_{k+1}\rangle$ in the (k+1)-th step expressed by following Formula (D) is set on a basis of the plurality of operators $P_{n1k},\cdots,P_{nsk}$ and the rotation angles $\theta_{1k}^{*},\cdots,\theta_{sk}^{*}$, and
the energy $E_{k+1}$ and the state vector $c_{k+1}$ obtained by executing the QSCI algorithm on a basis of the Hamiltonian H and the input state $|\psi_{k+1}\rangle$ in the (k+1)-th step are acquired.

[Formula 1]

$$\left\langle c_k \middle| e^{-i\theta_{s_k}P_{ns_k}} \cdots e^{-i\theta_{1_k}P_{n1_k}} H e^{i\theta_{1_k}P_{n1_k}} \cdots e^{i\theta_{s_k}P_{ns_k}} \middle| c_k \right\rangle$$

(C)

[Formula 2]

$$\left|\psi_{k+1}\right\rangle = e^{i\theta_{1_k}^{*}P_{n1_k}} \cdots e^{i\theta_{s_k}^{*}P_{ns_k}} \left|\psi_k\right\rangle$$

(D)

8. The information processing program for quantum computing according to claim 4, wherein the convergence condition is whether or not a difference between the energy $E_k$ computed in the k-th step and the energy $E_{k+1}$ computed in the (k+1)-th step satisfies a threshold condition.

9. The information processing program for quantum computing according to claim 4, wherein the convergence condition is whether or not the absolute value $|\langle c_k|i(HP-PH)|c_k\rangle|$ of the differential computed in the k-th step satisfies a predetermined value condition.

**10.** The information processing program for quantum computing according to any one of claims 4 to 9, wherein

a quantum circuit for setting the input state is set by adding the operator $P_{nk}$ that is a rotation gate having the rotation angle $\theta_k{}^*$ to the quantum circuit set in the k-th step, and

the QSCI algorithm is executed using the quantum circuit.

**11.** An information processing device for quantum computing that

acquires an energy $E_k$ and a state vector $c_k$, which are results obtained by executing a quantum-selected configuration interaction (QSCI) algorithm on a basis of a Hamiltonian H and an input state of a k-th step of qubits; and

sets an input state of a (k+1)-th step of the qubit on a basis of an energy expectation value according to the Hamiltonian H, the state vector $c_k$, and an operator P for each of operators P in a set of operators $\{P_1, P_2, \cdots, P_T\}$ set in advance.

**12.** An information processing system for quantum computing that

acquires an energy $E_k$ and a state vector $c_k$, which are results obtained by executing a quantum-selected configuration interaction (QSCI) algorithm on a basis of a Hamiltonian H and an input state of a k-th step of qubits; and

sets an input state of a (k+1)-th step of the qubit on a basis of an energy expectation value according to the Hamiltonian H, the state vector $c_k$, and an operator P for each of operators P in a set of operators $\{P_1, P_2, \cdots, P_T\}$ set in advance.

FIG.1

100

110

111

112

113

120

121

122

123

130

FIG.2

FIG.3

EP 4 752 793 A1

QUANTUM
COMPUTER

R BIT STRINGS WITH
HIGH APPEARANCE
FREQUENCY
$\{|0011\rangle, |0101\rangle, ...\}$

CLASSICAL
COMPUTER

INPUT
STATE $|\Psi\rangle$ →

COMPUTATIONAL
BASIS SAMPLING

→

DIAGONALIZE
HAMILTONIAN IN
SUBSPACE

→ STATE AND
ENERGY

## FIG.4

```
┌──────────────┐          ┌──────────┐       ┌──────────────┐
│    USER      │─130      │  SERVER  │─110   │   QUANTUM    │─120
│  TERMINAL    │          │          │       │  COMPUTER    │
└──────────────┘          └──────────┘       └──────────────┘
```

┌─────────────────────────────────┐
│ TRANSMIT COMPUTING TARGET        │─S100
│ INFORMATION AND COMPUTING        │
│ METHOD INFORMATION               │
└─────────────────────────────────┘

┌──────────────────────────────────────┐
│ RECEIVE COMPUTING TARGET INFORMATION  │─S102
│ AND COMPUTING METHOD INFORMATION      │
└──────────────────────────────────────┘

┌──────────────────────────────────────┐
│              $k \leftarrow 0$          │─S104
└──────────────────────────────────────┘

┌──────────────────────────────────────┐
│          SET FIRST INPUT STATE        │─S106
└──────────────────────────────────────┘

┌──────────────────────────────────────┐
│     TRANSMIT VARIOUS PIECES OF         │─S108
│            INFORMATION                 │
└──────────────────────────────────────┘

┌──────────────────────────────────────┐
│        EXECUTE QSCI ALGORITHM          │─S110
└──────────────────────────────────────┘

┌──────────────────────────────────────┐
│   ACQUIRE ENERGY $E_k$ AND STATE       │─S112
│           VECTOR $c_k$                 │
└──────────────────────────────────────┘

◇ IS CONVERGENCE CONDITION SATISFIED? ◇ ─S114

N

┌──────────────────────┐
│    SELECT OPERATOR    │─S116
└──────────────────────┘

┌──────────────────────┐
│   COMPUTE ROTATION    │─S118
│        ANGLE          │
└──────────────────────┘

┌──────────────────────┐
│    SET SECOND         │─S120
│    INPUT STATE        │
└──────────────────────┘

┌──────────────────────┐
│    $k \leftarrow k+1$ │─S122
└──────────────────────┘

Y

┌──────────────────────┐
│   TRANSMIT RESULT     │─S124
└──────────────────────┘

┌──────────────────────┐
│    RECEIVE RESULT     │─S126
└──────────────────────┘

┌────────┐      ┌────────┐      ┌────────┐
│  END   │      │  END   │      │  END   │
└────────┘      └────────┘      └────────┘

# FIG.5

## FIG.6

## INTERNATIONAL SEARCH REPORT

International application No.

**PCT/JP2023/037302**

| A. | CLASSIFICATION OF SUBJECT MATTER |
|---|---|

*G06N 10/60*(2022.01)i; *G16C 10/00*(2019.01)i
FI: G06N10/60; G16C10/00

According to International Patent Classification (IPC) or to both national classification and IPC

| B. | FIELDS SEARCHED |
|---|---|

Minimum documentation searched (classification system followed by classification symbols)

G06N10/00-10/80; G16C10/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KANNO, Keita et al. Quantum-Selected Configuration Interaction: classical diagonalization of Hamiltonians in subspaces selected by quantum computers. arXiv.org [online]. arXiv:2302.11320v1. Cornell University. February 2023, pp. 1-28, [retrieved on 01 December 2023], Internet:<URL: https://arxiv.org/abs/2302.11320v1> <br> abstract, chapters I-II, fig. 1 | 1-12 |
| A | GRIMSLEY, Harper R. et al. An adaptive variational algorithm for exact molecular simulations on a quantum computer. arXiv.org [online]. arXiv:1812.11173v2. Cornell University. 2019, pp. 1-11, [retrieved on 01 December 2023], Internet:<URL: https://arxiv.org/abs/1812.11173v2> <br> abstract, chapters I, II.B, fig. 1 | 1-12 |

☐ Further documents are listed in the continuation of Box C. ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 December 2023** | **12 December 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022026735 W

**Non-patent literature cited in the description**

- **A. PERUZZO** ; **J. MCCLEAN** ; **P. SHADBOLT** ; **M.-H. YUNG** ; **X.-Q. ZHOU** ; **P. J. LOVE** ; **A. ASPURU-GUZIK** ; **J. L. O'BRIEN**. A variational eigenvalue solver on a photonic quantum processor. *Nature Communications*, 2014, vol. 5, 4213
- **K. KANNO** ; **M. KOHDA** ; **R. IMAI** ; **S. KOH** ; **K. MITARAI** ; **W. MIZUKAMI** ; **Y. O. NAKAGAWA**. Quantum-selected configuration interaction: classical diagonalization of hamiltonians in subspaces selected by quantum computers. *arXiv preprint arXiv: 2302.11320*, 2023
- **K. KANNO** ; **M. KOHDA** ; **R. IMAI** ; **S. KOH** ; **K. MITARAI** ; **W. MIZUKAMI** ; **Y. O. NAKAGAWA**. Quantum-selected configuration interaction: classical diagonalization of hamiltonians in subspaces selected by quantum computers. *arXiv preprint arXiv:2302.11320*, 2023
- **C. DAVID SHERRILL** ; **HENRY F. SCHAEFER III**. The Configuration Interaction Method: Advances in Highly Correlated Approaches. *Advances in Quantum Chemistry*, 1999, vol. 34, 143-269
- **KEN M. NAKANISHI** ; **KEISUKE FUJII** ; **SYNGE TODO**. Sequential minimal optimization for quantum-classical hybrid algorithms. *Phys. Rev. Research*, 29 October 2020, vol. 2, https://journals.aps.org/prresearch/abstract/10.1103/PhysRevResearch.2.043158
- **H. R. GRIMSLEY** ; **S. E. ECONOMOU** ; **E. BARNES** ; **N. J. MAYHALL**. An adaptive variational algorithm for exact molecular simulations on a quantum computer. *Nature Communications*, 2019, vol. 10
- **H. L. TANG** ; **V. SHKOLNIKOV** ; **G. S. BARRON** ; **H. R. GRIMSLEY** ; **N. J. MAYHALL** ; **E. BARNES** ; **S. E. ECONOMOU**. Qubit-adapt-vqe: An adaptive algorithm for constructing hardware-efficient ansatze on a quantum processor. *PRX Quantum*, 2021, vol. 2, 020310
- **ABHINAV ANAND et al.** A quantum computing view on unitary coupled cluster theory. *Chemical Society Reviews*, 2022 (5), https://pubs.rsc.org/en/content/articlelanding/2022/CS/D1 CS00932J
- **TUDOR GIURGICA-TIRON et al.** *Digital zero noise extrapolation for quantum error mitigation*, https://ieeexplore.ieee.org/document/9259940
- **FILIP B. MACIEJEWSKI et al.** *Mitigation of readout noise in near-term quantum devices by classical postprocessing based on detector tomography*, https://quantum-journal.org/papers/q-2020-04-24-257